Europäisches Patentamt

(19) European Patent Office

Office européen des brevets

(11) Numéro de publication : **0 437 500 B1**

(12) **FASCICULE DE BREVET EUROPEEN**

(45) Date de publication du fascicule du brevet :
**14.07.93 Bulletin 93/28**

(51) Int. Cl.⁵ : **C12N 9/08**

(21) Numéro de dépôt : **89911570.3**

(22) Date de dépôt : **02.10.89**

(86) Numéro de dépôt international :
**PCT/FR89/00506**

(87) Numéro de publication internationale :
**WO 90/04021 19.04.90 Gazette 90/09**

(54) **PROCEDE DE PRODUCTION DE LIGNINE-PEROXYDASE PAR DES CELLULES NON-PROLIFERANTES DE -i(PHANEROCHAETE CHRYSOSPORIUM).**

(30) Priorité : **03.10.88 FR 8812887**

(43) Date de publication de la demande :
**24.07.91 Bulletin 91/30**

(45) Mention de la délivrance du brevet :
**14.07.93 Bulletin 93/28**

(84) Etats contractants désignés :
**AT BE CH DE FR GB IT LI LU NL SE**

(56) Documents cités :
**EP-A- 0 188 931**
**FR-A- 2 600 077**
**Journal of Biotechnology, vol. 8, no.2, juin 1988, Elsevier Science Publishers B.V. (Amsterdam,NL) S. Linko "Continuous production of lignin peroxidase by immobilized phanerochaete chrysosporium in a pilot scale bioreactor", pages 163-170**
**Fems Microbiology Letters, vol. 35, 1986, Elsevier. S.D. Haemmerli et al "Polymerisation of lignins by ligninases from phanerochaete chrysosporium", pages 33-36**
**Chemical Abstracts, vol. 106, 1987, (Columbus, Ohio, US) M. Asther et al "Effect of tween 80 and oleic acid on ligninase producton by phanerochaete chrysosporium INA-12", voir page 600, résumé 194719s & Enzyme Microb, Technol. 1987 9(4), 245-9**
**Chemical Abstracts, vol. 109, 1988, (Columbus, Ohio, US) M. Asther et al "Strategies to enhanced ligninase production by phanerochaete chrysosporium", voir page 484, résumé 108861q & Fems Symp. 1988, 43 (Biochem. Genet. Cellul. Degrad), 333-47**

(56) Documents cités :
**Chemical Abstracts, vol. 109, 1988 (Columbus, Ohio, US) F. Tohon et al "Influence of medium additives for the production of active ligninase by phanercchaete chrysosporium", voir page 484, résumé 108857t & Colloq. Inra, 1987, 40 (Lignin enzymic Microb. Degrad) 165-170**
**Chemical Abstracts, vol. 108, 1988 (Columbus, Ohio, US) M. Asther et al "Phospholipid and fatty acid enrichment of phanerochaete chrysosporium INA-12 in relation to ligninase production", voir page 541, résumé 73699t & Appl. Microbiol. Biotechnol. 1988, 27(4), 393-8**
**Biological Abstracts, Annual Meeting of the American Society for Microbiology, Florida, 8-13 mai 1988. M. Asther et al "Improvement by temperature-shifting of ligninase production by phanerochaete chrysospopium INA-12", voir résumé BR35:48606 & Abstr. Annu. Meet. Am. Soc. Microbiol. 88 (O), 1988, 264**

(73) Titulaire : **INSTITUT NATIONAL DE LA RECHERCHE AGRONOMIQUE**
**145, rue de l'Université**
**F-75341 Paris Cédex 07 (FR)**

(72) Inventeur : **ASTHER, Marcel**
**1, allée d'Auxois**
**F-78310 Maurepas (FR)**
Inventeur : **CAPDEVILA, Cécile**
**5, rue des Chantiers**
**F-78000 Versailles (FR)**
Inventeur : **CORRIEU, Georges**
**2, avenue des Combattants**
**F-78220 Viroflay (FR)**

(74) Mandataire : **Orès, Bernard et al**
**Cabinet ORES 6, Avenue de Messine**
**F-75008 Paris (FR)**

Jouve, 18, rue Saint-Denis, 75001 PARIS

## Description

La présente invention est relative à un procédé perfectionné de production d'enzyme ligninolytique ou lignine-peroxydase.

MING TIEN et T. KENT KIRK ont décrit dans SCIENCE, (1983), 221, p.661-663, une enzyme extracellulaire produite par *Phanerochaete chrysosporium* Burdsall qui est un champignon basidiomycète ; cette enzyme est capable de provoquer en présence de peroxyde d'hydrogène, la dégradation oxydante de différents composés modèles présentant des sous-structures de la lignine, de même que la dégradation de la lignine de sapin ou de bouleau.

Cette enzyme, dénommée " lignine-peroxydase ", a été caractérisée [M. TIEN et T.K. KIRK PROC. NATL. ACAD. SCI. USA, (1984) vol. 81, p. 2280-2284] comme étant une glycoprotéine d'environ 42KDa.

Le brevet Français 2 574 427 revendique deux nouvelles souches de *Phanerochaete chrysosporium* Burdsall capables de développer, dans des milieux non limités en azote, une activité lignolytique particulièrement élevée, alors que les souches précédemment connues ne produisaient l'enzyme ligninolytique qu'en milieu de culture carencé en azote, le processus de biodégradation produisant cette enzyme étant, de surcroit très lent, dans ces conditions.

Selon ce brevet, le milieu de culture propre à favoriser la production de lignine-peroxydase contient une source d'azote assimilable qui peut être l'asparagine, le nitrate d'ammonium ou le tartrate d'ammonium ; il contient également une source de carbone assimilable telle que glycose, mannose, amidon, mélibiose, mannitol, xylose, maltose, adonitol, arabitol, fructose, sorbitol, raffinose, xylitol, D(+)trehalose, glycérol, mais de préférence ce dernier ; il contient en outre une source de sels minéraux assimilables tels que citrate de fer, $KH_2PO_4$, $ZnSO_4$, $MnSO_4$, $CaCl_2$, $CuSO_4$, $NaCl$, $FeSO_4$, $CoCO_4$, $AlK(SO_4)_2$, $H_3BO_3$, $Na_2MoO_4$, $MgSO_4$.

Le brevet Français 2 600 077 propose d'augmenter de façon significative les rendements de production de lignine-peroxydase, en cultivant le champignon *Phanerochaete chrysosporium* sur un milieu de culture de base supplémenté avec un agent stimulant choisi parmi les acides gras insaturés, les acides aminés naturels et leurs mélanges. Les acides gras insaturés préférés sont l'acide oléique, l'acide linoléique, l'acide palmitoléique, l'acide arachinolique, de préférence émulsifiés, et les acides aminés naturels préférés sont la sérine, la thréonine, l'isoleucine, la glycine, la valine, la tyrosine, mais plus particulièrement l'isoleuciné, la sérine, la thréonine et la glycine. La concentration optimale d'acide oléique est d'environ 800 mg/litre, et elle est d'environ 400 mg/litre lorsqu'il s'agit d'acide oléique émulsifié ; elle est d'environ 6,5 mg/litre pour l'isoleucine.

Il faut rappeler que JÄGER et Al. (APPLIED AND ENVIRONMENTAL MICROBIOLOGY, Nov. 1985, p.1274-1278) avaient proposé une addition de détergent tel que TWEEN 80 ou TWEEN 20 au milieu de culture du champignon, dans les cultures submergées, pour augmenter la production de lignine-peroxydase dans des proportions comparables à celle couramment obtenue en cultures stationnaires. FAISON & KIRK [APPL. ENVIRON. MICROBIOL. (1985), 49, p. 299-304] et LEISOLA et Al. [J.BIOTECHNOL. (1985), 3, p. 97-107] ont rapporté que l'addition d'alcool vératrylique, qui est un métabolite secondaire de *Phanerochaete chrysosporium,* augmente la synthèse de la lignine-peroxydase.

Dans un article paru dans ENZ. MICROBIAL TECHNOL. (1987), 9, p.245-249, ASTHER et Al. ont montré que la production de lignine-peroxydase a été augmentée de façon significative et la durée de fermentation jusqu'à obtention de l'activité enzymatique maximale, fortement réduite, en présence d'acide oléique exogène émulsifié par du TWEEN 80.

Dans un article plus récent, paru dans APPL. MICROBIOL. BIOTECHNOL. (1988) 27,p.393-398, ASTHER et Al. ont rapporté que la production de lignine-peroxydase par le champignon *Phanerochaete chrysosporium,* est encore augmentée lorsqu'on ajoute au milieu de culture, de l'huile d'olive supplémentée avec de l'azolectine de soja qui constitue une source de phospholipides.

Dans une Communication faite lors de la Rencontre annuelle de l'ASM qui a eu lieu du 8 au 13 Mai 1988 à Miami, Floride, Etats-Unis, ASTHER et Al. ont rapporté que l'on obtient une production maximale de lignine-peroxydase à partir de cultures de *Phanerochaete chrysosporium* lorsque la culture est réalisée à deux températures différentes, à savoir à 37°C pendant la phase de croissance du mycelium, pendant les deux premiers jours de l'incubation, puis à 30°C pendant la phase de production de lignine-peroxydase.

LEISOLA et Al. [cf. l'Article cité plus haut paru en 1985 dans J. BIOTECHNOL.] ont identifié quatre hèmes contenant de l'activité lignine-peroxydase, dans du fluide extracellulaire de culture âgées de trois jours de *Phanerochaete chrysosporium* BKM-F-1767, induites par de l'alcool vératrylique. JÄGER et Al. [APPL. ENVIRON. MICROBIOL. (1985) 50, p.1274-1278] ont séparé 8 pics présentant une absorbance de 409 nm (hèmeprotéines) et ont supposé que des différences relativement mineures entre les profils des protéines reflètent simplement l'âge des cultures.

La présente invention s'est donné pour but de pourvoir à un procédé de production de lignine-peroxydase à partir de *Phanerochaete chrysosporium* dans des conditions de température contrôlées différentes pendant la phase de croissance du mycelium et pendant la phase de production de lignine-peroxydase proprement dite, qui permettent de privilégier la production des hèmeprotéines présentant l'activité lignine-peroxydase maximale, au détriment de la production de biomasse.

La présente invention a pour objet un procédé de production de lignine-peroxydase à partir d'un champignon connu sous le nom de *Phanerochaete chrysosporium*, dans un milieu de culture contenant des activateurs de l'enzyme, tels que des phospholipides et de l'alcool vératrylique, lequel procédé est caractérisé en ce qu'il comprend :

- une première étape de culture de cellules de *Phanerochaete chrysosporium* pendant une période d'incubation de 2 jours environ, dans un milieu de culture synthétique comprenant des sels de potassium, de calcium et de magnésium, des oligo-éléments (fer, zinc, manganèse, cuivre), une source d'azote appropriée, une source de carbone avantageusement constituée par du glycérol, de l'extrait de levure, une source de phospholipides, une source d'acide gras émulsifiés ;
- une deuxième étape de culture du mycelium formé au cours de la première étape, pendant une période de 3 jours environ (du J3 au J5), dans un milieu de culture en partie renouvelé (avantageusement à raison de 15% environ), additionné d'alcool vératrylique, activateur et protecteur de la production de la lignine-peroxydase, et dont la teneur en phospholipides est réduite au 1/7ème - 1/8ème environ de sa teneur dans le milieu de culture de la première étape, lequel milieu de culture ne contient pas d'acides gras émulsifiés ;
- une troisième étape de culture, à l'optimum de production de la lignine-peroxydase, c'est-à-dire au bout de 5 jours environ, au cours de laquelle la totalité du milieu de culture de la deuxième étape est remplacée par un milieu de culture synthétique analogue à celui de la première étape, contenant la même proportion de phospholipides et d'alcool vératrylique que le milieu de culture de la deuxième étape et dans lequel l'extrait de levure, la source d'azote et la source de carbone - avantageusement constituée par du glycérol - sont réduits au 1/4 de leur teneur dans le milieu de culture de la première étape, ces trois composants constituant en combinaison un milieu de régénération partielle de la biomasse ;
- une quatrième étape qui consiste à poursuivre la culture avec des cellules non proliférantes pendant 8 jours environ (de J6 à J13), en renouvelant totalement le milieu de culture toutes les 24 heures pour le remplacer par un milieu ne contenant ni extrait de levure, ni acides gras émulsifiés, ni glycérol, mais contenant les activateurs et protecteurs de la production de l'enzyme constitués par l'alcool vératrylique et les phospholipides dans les mêmes proportions que les milieux de culture des deuxième et troisième étapes ;
- une cinquième étape de séparation de l'enzyme produite et éventuellement de purification de celle-ci.

Selon un mode de mise en oeuvre avantageux du procédé de production de lignine-peroxydase conforme à la présente invention, la première étape de culture des cellules a lieu à une température d'incubation de 37°C environ, alors que les étapes suivantes sont réalisées à des températures d'incubation de 30°C environ.

Selon un autre mode de mise en oeuvre avantageux du procédé conforme à la présente invention, la première étape de culture des cellules a lieu dans un milieu de culture tamponné à pH 6,5, alors que les étapes suivantes sont réalisées dans un milieu de culture tamponné à pH 5,5;

Selon encore un autre mode de mise en oeuvre avantageux du procédé de production de lignine-peroxydase conforme à la présente invention, la source de phospholipides est avantageusement constituée par de l'azolectine de soja.

Selon un autre mode de mise en oeuvre avantageux du procédé de production de lignine-peroxydase conforme à la présente invention, le milieu de culture de la première étape comprend une teneur en source d'azote de l'ordre de 1,84 g/litre pour une teneur en source de carbone (glycerol) de l'ordre de 10 g/litre, pour une teneur en phospholipides (azolectine de soja) de l'ordre de 0,75 g/litre et pour une teneur en extrait de levure de 1 g/litre.

Selon encore un autre mode de mise en oeuvre avantageux du procédé de production de lignine-peroxydase conforme à la présente invention, le milieu de culture de la deuxième étape diffère du milieu de culture de la première étape par son pH (5,5), sa teneur réduite en phospholipides (de l'ordre de 0,1 g/litre) et sa teneur en alcool vératrylique (de l'ordre de 2,5 mM).

Selon un autre mode de mise en oeuvre avantageux du procédé de production de lignine-peroxydase conforme à la présente invention, le milieu de culture de la troisième étape diffère du milieu de culture de la deuxième étape, par sa teneur réduite en glycérol (2,5 g/litre environ), en source d'azote (notamment 0,46 g/litre environ de tartrate de diammonium) et en extrait de levure (0,25 g/litre).

Selon un autre mode de mise en oeuvre avantageux du procédé de production de lignine-peroxydase conforme à la présente invention, le milieu de culture de la quatrième étape diffère du milieu de culture de la troisième étape en ce qu'il ne contient ni extrait de levure, ni source d'azote, ni source de carbone.

Selon un autre mode de mise en oeuvre avantageux du procédé de production de lignine-peroxydase conforme à l'invention, les troisième et quatrième étapes sont réalisées en continu.

Le procédé conforme à la présente invention permet une augmentation considérable de la productivité en lignine-peroxydase et de l'activité lignine-peroxydase par rapport à l'ensemble des procédés connus à ce jour. En effet, alors que l'on obtient une activité lignine-peroxydase de l'ordre de 22,4 nKat. ml$^{-1}$ dans le cas où un milieu de culture de *Phanerochaete chrysosporium* est supplémenté en acide oléique émulsifié avec du Tween 80 à une concentration de 0,04 % (poids/volume) et alors que l'activité lignine-peroxydase est de l'ordre de 33,3 nKat. ml$^{-1}$ lorsque le milieu de culture est supplémenté avec de l'azolectine comme source de phospholipides et de l'huile d'olive comme source de lipides, l'activité lignine-peroxydase peut atteindre 40,6 nKat. ml$^{-1}$ lorsque l'on met en oeuvre le procédé conforme à la présente invention, dans lequel des milieux de culture supplémentés successivement en phospholipides et acides gras émulsifiés, puis en alcool vératrylique et phospholipides, sont utilisés à des températures d'incubation différentes, de 37°C pour le premier et de 30°C pour le second. En outre, alors que la productivité en lignine-peroxydase est de l'ordre de 40,6 nKat. ml$^{-1}$ . jour$^{-1}$ lorsqu'on met en oeuvre le procédé conforme à l'invention, elle est aussi faible que 4,5 nKat. ml$^{-1}$ . jour$^{-1}$ lorsque le milieu de culture contient de l'acide oléique émulsifié avec du Tween 80, elle est de 8,3 nKat. ml$^{-1}$. j$^{-1}$ lorsque le milieu de culture contient de l'azolectine et de l'huile d'olive et elle est de 1,6 nKat. ml$^{-1}$ .jour$^{-1}$ lorsque le milieu de culture contient de l'alcool vératrylique 1mM, avec une température d'incubation de 39°C comme décrit dans l'Art antérieur cité.

Les profils de chromatographie FPLC (fast protein liquid chromatography) de protéines extracellulaires obtenues à partir de cultures incubées à 37°C pendant les deux premiers jours, puis à 30°C, avec les changements de milieux de culture conformes à la présente invention montrent la présence de plusieurs hèmeprotéines dont neuf présentent une activité lignine-peroxydase, avec un grand pic N°4.

Outre les dispositions qui précèdent, l'invention comprend encore d'autres dispositions, qui ressortiront de la description qui va suivre.

L'invention sera mieux comprise à l'aide du complément de description qui va suivre, qui se réfère aux dessins annexés dans lesquels :

la figure 1 représente le schéma de déroulement du procédé conforme à la présente invention et

la figure 2 représente les profils FPLC des isoenzymes de la lignine-peroxydase au bout de 5 jours de culture, en fonction des conditions de culture.

Il doit être bien entendu, toutefois, que ces dessins et les parties descriptives correspondantes, sont donnés uniquement à titre d'illustration de l'objet de l'invention, dont ils ne constituent en aucune manière une limitation.

EXEMPLE 1 DE PRODUCTION DE LIGNINE-PEROXYDASE

Le microorganisme mis en oeuvre est la souche *Phanerochaete chrysosporium* INA-12 (CNCM N°I-398).

Des cellules de *Phanerochaete chrysosporium* INA-12 immobilisées sur de la mousse de polyuréthane sont traitées comme suit :

1. On inocule 6 x 10$^7$ conidiospores dans un milieu de culture présentant la composition suivante :

## Solution de sels

| | | |
|---|---|---|
| • Potassium dihydrogénophosphate | $KH_2PO_4$ | 2 g/l |
| • chlorure de calcium | $CaCl_2$, $2H_2O$ | 0,14 g/l |
| • Sulfate de magnesium | $Mg\ SO_4$, $7H_2O$ | 0,7 g/l |

## Solution d'oligoéléments

| | | |
|---|---|---|
| • Sulfate de fer (ferreux) | $FeSO_4$, $7H_2O$ | 0,07 g/l |
| • Sulfate de zinc | $ZnSO_4$, $7H_2O$ | 0,0462 g/l |
| • Sulfate de manganèse | $MnSO_4$, $H_2O$ | 0,035 g/l |
| • Sulfate de cuivre | $CuSO_4$, $5H_2O$ | 0,007 g/l |

<u>Solution de Vitamine</u>

. chlorydrate de Thiamine (vitamine $B_1$)          0,0025 g/l

<u>Source d'azote</u>

. Tartrate d'ammonium          1,84 g/l

<u>Source de carbone</u>

. glycérol          10 g/l

. extrait de levure          1 g/l

. azolectine de soja (phospholipides)          0,75 g/l

. acide oléique émulsifié avec du Tween 80          0,4 g/l

<u>pH de la culture</u> : pH : 6,5 (le tampon utilisé est l'acide 2,2-diméthyl-succinique)

<u>Température d'incubation</u> : 37°C

La culture est réalisée après insufflation de 100% d'$O_2$ pendant deux minutes, sans agitation, dans des Erlenmayer de 150 ml contenant 30 ml du milieu de culture aqueux susdit, de J0 à J2 à 37°C.

2. On soutire 15% du milieu total susdit, qu'on remplace par un milieu de culture tel que décrit en 1. ci-dessus sauf en ce qui concerne l'extrait de levure, l'azolectine de soja et l'acide oléique émulsifié dont le premier et le troisième sont supprimés, de même que le glycerol, le tartrate de diammonium, les sels, les oligoéléments et les vitamines, tandis que la teneur en azolectine de soja du milieu est diminuée de 7,5 fois (0,1 g/litre) et que ledit milieu est additionné d'alcool vératrylique (2,5 mM), l'alcool vératrylique et l'azolectine de soja jouant le rôle d'inducteurs de production de la lignine-peroxydase.

La culture est poursuivie de J3 à J5 inclus à une température d'incubation de 30°C, qui est la température optimale de production de lignine-peroxydase.

3. à J5, la totalité du milieu de culture est soutirée pour être remplacée par un milieu de culture présentant la composition suivante :

source d'azote

tartrate de diammonium     0,46 g/l

source de carbone

glycérol          2,5 g/l

extrait de levure          0,25 g/l

azolectine de soja          0,1 g/l

alcool vératrylique          2,5 mM

L'alcool vératrylique et l'azolectine de soja présents dans ce milieu jouent le rôle d'inducteurs et de protecteurs de la lignine-peroxydase, comme dans le milieu de culture utilisé dans l'étape 2. ci-dessus et le glycérol, le tartrate d'ammonium et l'extrait de levure jouent en combinaison, le rôle de milieu de régénération de la biomasse. Le pH du milieu de culture est ajusté à pH 5,5 et la température d'incubation est de 30°C. Le glycérol, la source d'azote et l'extrait de levure agissent sur la production de biomasse et non sur la production de l'enzyme ; c'est pourquoi ils sont présents dans le milieu de régénération. Toutefois leurs concentrations sont réduites à 25% de ce qu'elles sont dans le milieu de culture de l'étape 1. ci-dessus pour limiter la production de biomasse dans des proportions non préjudiciables à la production de l'enzyme.

4. A partir de J6 la totalité du milieu de culture est soutirée toutes les 24 heures pour être remplacée par un milieu de culture de même composition que celui utilisé dans l'étape 2. ci-dessus, et la culture est poursuivie jusqu'à J13, à 30°C à pH 5,5.

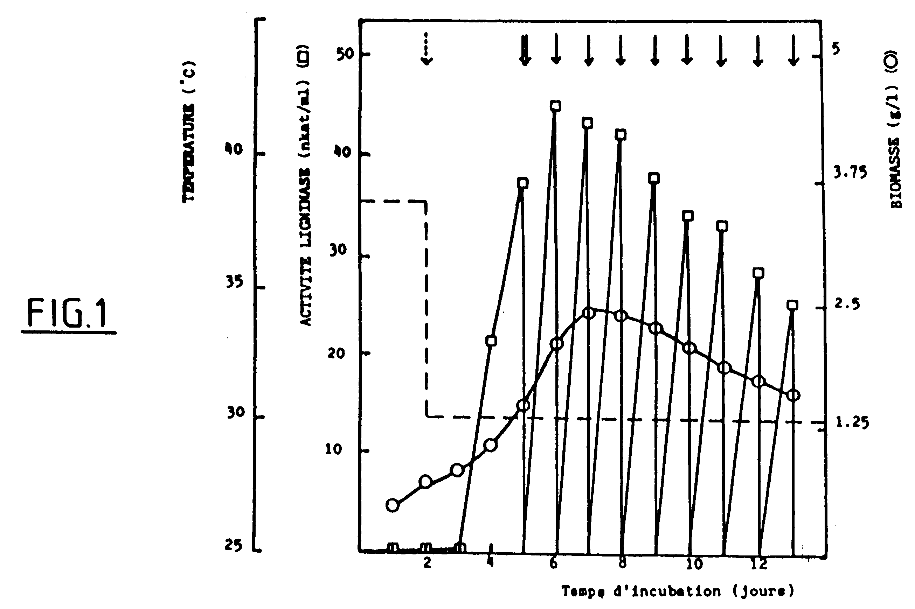

La figure 1 annexée constitue un schéma du procédé de production de la lignine-peroxydase par des cellules non-proliférantes de *Phanerochaete chrysosporium*, conforme à la présente invention.

Dans ce schéma :

_ _ _ _ _ représente le profil de température

représente le remplaçement de 15% du milieu de culture par un milieu de culture contenant 0,1 g/l d'azolectine et 2,5 mM d'alcool vératrylique

représente le remplacement de 100% du milieu de culture par un milieu de culture contenant de l'azolectine (0,1 g/l), de l'alcool vératrylique (2,5 mM) et un milieu de régénération partielle de la biomasse (glycérol 2,5 g/l, tartrate d'ammonium 0,46 g/l , extrait de levure 0,25 g/l)

représente le remplacement (toutes les 24 heures) de 100% du milieu de culture par un milieu de culture contenant de l'azolectine (0,1 g/l) et de l'alcool vératrylique (2,5 mM).

5. Les cultures obtenues sont récoltées et filtrées sur un filtre en fibres de verre. Le surnageant est dialysé pendant une nuit contre de l'eau distillée. Le milieu extracellulaire est alors concentré au 1/10ème de son volume initial par ultrafiltration à travers une membrane Amicon YM10.

Les protéines contenues dans le fluide concentré sont déterminées par chromatographie FPLC (fast protein liquid chromatography) à l'aide d'un chromatographe LCC 500 de PHARMACIA équipé d'une colonne échangeuse d'anions Mono QHR 515, avec un gradient de NaCl dans 10 mM de cacodylate de sodium (pH 5,9).

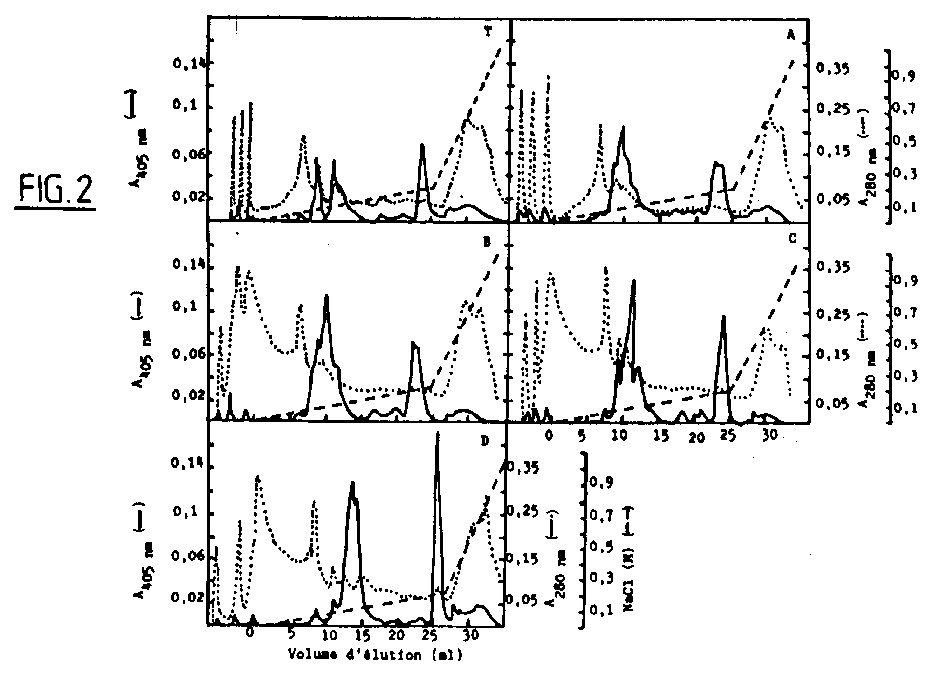

Les profils FPLC des isoenzymes de la lignine-peroxydase obtenus sont représentés à la figure 2 annexée dans laquelle

la figure 2A représente les profils de protéines extracellulaires de *Phanerochaete chrysosporium* INA-12 dans les conditions de culture standard sans renouvellement du milieu de culture, la température d'incubation étant de 37°C ;

la figure 2B représente lesdits profils de protéines obtenus avec renouvellement de l'atmosphère (100% d'oxygène) après deux jours de culture

la figure 2C représente lesdits profils de protéines obtenus avec renouvellement de l'atmosphère en oxygène et changement de 15% du milieu avec 2,5mM d'alcool vératrylique après deux jours de culture

la figure 2D représente lesdits profils de protéines obtenus avec renouvellement de l'atmosphère en oxygène et changement de 15% du milieu avec 2,5mM d'alcool vératrylique et 0,1 g/l d'azolectine après deux jours de culture

la figure 2E représente lesdits profils de protéines obtenus avec renouvellement de l'atmosphère en oxygène et changement de 15% du milieu avec 2,5 mM d'alcool vératrylique et 0,1 g/l d'azolectine et changement de température d'incubation de 37°C à 30°C après deux jours de culture. L'absorbance à 405 nm est représentée en traits pleins et l'absorbance à 280 nm en traits discontinus ; la droite inclinée représente le gradient de NaCl.

Ces profils montrent que le fluide extracellulaire contient plusieurs hèmeprotéines dont six présentent une activité lignine-peroxydase ; ce sont les pics 1, 2, 3, 4, 5, 6, 7, 8 et 9. 80% de l'activité lignine-peroxydase sont associés aux pics 7, 8 et 9. Les cultures dont les températures d'incubation ont été successivement de 37°C, puis de 30°C, et celles dont la température d'incubation a été uniquement de 30°C présentent une augmentation de la proportion du pic 4.

EXEMPLE 2 DE PRODUCTION DE LIGNINE-PEROXYDASE

On reproduit les conditions de l'exemple 1 en introduisant dans un Erlenmayer de 250 ml, 100 ml de milieu ; on obtient une production d'enzyme de 46,4 nKat /ml.

EXEMPLE 3 DE PRODUCTION DE LIGNINE-PEROXYDASE

Le microorganisme mis en oeuvre est la souche *Phanerochaete chrysosporium* INA-12 (CNCM N°I-398).

Des cellules de *Phanerochaete chrysosporium* INA-12 immobilisées sur de la mousse de polyuréthane sont traitées comme dans l'étape 1 de l'exemple 1.

Le réacteur est ensemencé avec $2.10^5$ spores/ml. Il y a une immobilisation directe *in situ* dans le réacteur.

Le milieu est alimenté en continu en oxygène, introduit dans une cheminée centrale où est également situtée la turbine d'agitation (200 t/mn). La concentration en $O_2$ est régulée aux environs de 60 % de la saturation du milieu par l'air. Les cellules de *Phanerochaete chrysosporium* immobilisées sur la mousse de polyuréthane sont disposées à la périphérie du bioréacteur, autour de la cheminée centrale.

Les étapes 2 à 5 sont identiques à celles de l'exemple 1.

Le titre en enzyme obtenue atteint 25 nKat/ml.

Le procédé conforme à la présente invention permet de contrôler la production de lignine-peroxydase et, par voie de conséquence, la possibilité d'une production de cette enzyme à l'échelle industrielle.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

**Revendications**

1. Procédé de production de lignine-peroxydase à partir d'un champignon connu sous le nom de *Phanerochaete chrysosporium*, dans un milieu de culture contenant des activateurs et protecteurs de l'enzyme, tels que des phospholipides et de l'alcool vératrylique, lequel procédé est caractérisé en ce qu'il comprend :
   - une première étape de culture de cellules de *Phanerochaete chrysosporium* pendant une période d'incubation de 2 jours, dans un milieu de culture synthétique comprenant des sels de potassium, de calcium et de magnésium, des oligo-éléments (fer, zinc, manganèse, cuivre), une source d'azote appropriée, une source de carbone constituée par du glycérol, de l'extrait de levure, une source de phospholipides, une source d'acides gras émulsifiés, laquelle première étape a lieu à une température d'incubation de 37°C ± 0,5°C, le milieu de culture étant tamponné à pH 6,5 ;
   - une deuxième étape de culture du mycelium formé au cours de la première étape, pendant une période de 3 jours (de J3 au J5) dans un milieu de culture en partie renouvelé (de préférence renouvelé à raison de 15%), additionné d'alcool vératrylique, activateur et protecteur de la production de la lignine-peroxydase, conjointement avec les phospholipides, et dont la teneur en phospholipides est réduite au 1/7ème - 1/8ème de sa teneur dans le milieu de culture de la première étape, lequel milieu de culture ne contient pas d'acides gras émulsifiés et est tamponné à pH 5,5, cette deuxième étape se déroulant à une température d'incubation de 30°C ± 0,5°C ;
   - une troisième étape de culture, à l'optimum de production de la lignine-peroxydase, c'est-à-dire au bout de 5 jours, au cours de laquelle la totalité du milieu de culture de la deuxième étape est remplacée par un milieu de culture synthétique analogue à celui de la première étape contenant la même proportion de phospholipides et d'alcool vératrylique que le milieu de culture de la deuxième étape et dans lequel l'extrait de levure, la source d'azote et la source de carbone - constituée par du glycérol - sont réduits au 1/4 de leur teneur dans le milieu de culture de la première étape, ces trois composants constituant en combinaison un milieu de régénération partielle de la biomasse, la température d'incubation étant de 30°C ± 0,5°C et le pH du milieu de culture étant de pH 5,5 ;
   - une quatrième étape qui consiste à poursuivre la culture avec des cellules non proliférantes pendant 8 jours (de J6 à J13), en renouvelant totalement le milieu de culture toutes les 24 heures pour le remplacer par un milieu ne contenant ni extrait de levure, ni acides gras émulsifiés, ni glycérol, mais contenant des activateurs de production et de production de l'enzyme constitués par l'alcool vératrylique et les phospholipides dans les mêmes proportions que les milieux de culture des deuxième et troisième étapes, le pH et la température d'incubation étant les mêmes qu'au cours des deuxième et troisième étapes ;
- une cinquième étape de séparation de l'enzyme produite et éventuellement de purification de celle-ci.

2. Procédé selon la revendication 1, caractérisé en ce que la source de phospholipides est constituée par de l'azolectine de soja.

3. Procédé selon la revendication 1, caractérisé en ce que le milieu de culture de la première étape comprend une teneur en source d'azote de l'ordre de 1,84 g/litre pour une teneur en source de carbone (glycérol) de l'ordre de 10 g/litre, pour une teneur en phospholipides (azolectine de soja) de l'ordre de 0,75 g/litre et pour une teneur en extrait de levure de 1 g/litre.

4. Procédé selon l'une quelconque des revendications 1 à 3, caractérisé en ce que le milieu de culture de la deuxième étape diffère du milieu de culture de la première étape par son pH (5,5), sa teneur réduite en phospholipides (de l'ordre de 0,1 g/litre) et sa teneur en alcool vératrylique (de l'ordre de 2,5 mM).

5. Procédé selon l'une quelconque des revendications 1 à 4, caractérisé en ce que le milieu de culture de la troisième étape diffère du milieu de culture de la deuxième étape, par sa teneur réduite en glycérol (2,5 g/litre environ), en source d'azote (notamment 0,46 g/litre ± 0,04 g/l de tartrate de diammonium) et en extrait de levure (0,25 g/litre).

6. Procédé selon l'une quelconque des revendications 1 à 5, caractérisé en ce que le milieu de culture de la quatrième étape diffère du milieu de culture de la troisième étape en ce qu'il ne contient ni extrait de levure, ni source d'azote, ni source de carbone.

7. Procédé selon l'une quelconque des revendications 1 à 6, caractérisé en ce que les cultures obtenues sont récoltées et sont séparées par filtration, puis le milieu extracellulaire obtenu est concentré pour recueillir la lignine-peroxydase recherchée.


**Patentansprüche**

1. Verfahren zur Erzeugung einer Ligninperoxidase aus einem Pilz, der unter dem Namen Phanerochaete chrysosporium bekannt ist, in einem Kulturmilieu, das Aktivatoren und Schutzstoffe des Enzyms, wie Phospholipide und Veratrylalkohol, enthält, dadurch **gekennzeichnet,** daß es die folgenden Stufen umfaßt:
   - eine erste Stufe der Züchtung der Zellen von Phanerochaete chrysosporium während einer zweitägigen Inkubationsperiode in einem synthetischen Kulturmilieu mit Kalium-, Calcium- und Magnesiumsalzen, Spurenelementen (Eisen, Zink, Mangan, Kupfer), einer geeigneten Stickstoffquelle, Glycerin als Kohlenstoffquelle, Hefeextrakt, einer Quelle für Phospholipide, einer Quelle für emulgierte Fettsäuren, wobei diese erste Stufe bei einer Inkubationstemperatur von 37°C ± 0,5°C stattfindet, und das Kulturmilieu bei pH 6,5 gepuffert ist;
   - eine zweite Stufe der Züchtung des im Verlauf der ersten Stufe gebildeten Mycels während einer Zeitspanne von 3 Tagen (von T3 bis T5) in einem teilweise erneuerten Kulturmilieu (bevorzugt um 15% erneuert), welches mit Veratrylalkohol, einem Aktivator und Schutzstoff der Erzeugung der Ligninperoxidase zusammen mit Phospholipiden versetzt wurde, und dessen Gehalt an Phospholipiden auf ein Siebtel bis ein Achtel des Gehalts in dem Kulturmedium der ersten Stufe reduziert wurde, wobei das Kulturmedium keine emulgierten Fettsäuren enthält und auf pH 5,5 gepuffert ist, wobei diese zweite Stufe bei einer Inkubationstemperatur von 30°C ± 0,5°C stattfindet;
   - eine dritte Stufe der Züchtung beim Produktionsoptimum der Ligninperoxidase, d.h. nach 5 Tagen, in Verlauf derer das gesamte Kulturmilieu der zweiten Stufe durch ein dem der ersten Stufe analoges, synthetisches Kulturmilieu ersetzt wurde, welches das gleiche Verhältnis an Phospholipiden und Veratrylalkohol wie das Kulturmedium der zweiten Stufe enthält, und worin der Hefeextrakt die Stickstoffquelle und die Kohlenstoffquelle - bestehend aus Glycerin - auf ein Viertel ihres Gehalts in dem Kulturmedium der ersten Stufe verringert sind, wobei diese drei Komponenten in Kombination ein Teilregenerationsmilieu der Biomasse darstellen, wobei die Inkubationstemperatur 30°C ± 0,5°C beträgt, und der pH des Kulturmediums 5,5 beträgt;
   - eine vierte Stufe, die aus der Weiterzüchtung mit Zellen, die in den 8 Tagen nicht proliferiert hatten (von T6 bis T13), besteht, wobei das Kulturmedium alle 24 Stunden vollständig erneuert wird und gegen ein Milieu, das weder Hefeextrakt noch emulgierte Fettsäuren noch Glycerin enthält, sondern Aktivatoren zur Erzeugung und zum Schutz des Enzyms enthält, bestehend aus Veratrylalkohol und Phospholipiden in gleichen Anteilen wie in den Kulturmedien der zweiten und dritten Stufe, ausgetauscht wird, wobei der pH und die Inkubationstemperatur die gleichen sind wie im Verlauf der zweiten und dritten Stufe;
- eine fünfte Stufe der Abtrennung des erzeugten Enzyms und gegebenenfalls dessen Reinigung.

2. Verfahren nach Anspruch 1, dadurch **gekennzeichnet**, daß die Phospholipidquelle Sojaazolectin ist.

3. Verfahren nach Anspruch 1, dadurch **gekennzeichnet**, daß das Kulturmilieu der ersten Stufe einen Gehalt an der Stickstoffquelle in der Größenordnung von 1,84 g/Liter, bezogen auf einen Gehalt an der Kohlenstoffquelle (Glycerin) in der Größenordnung von 10 g/Liter, einen Gehalt an Phospholipiden (Sojaazolectin) in der Größenordnung von 0,75 g/Liter und einen Gehalt an Hefeextrakt von 1 g/Liter, enthält.

4. Verfahren nach einem der Ansprüche 1 bis 3, dadurch **gekennzeichnet,** daß das Kulturmilieu der zweiten Stufe sich von dem Kulturmilieu der ersten Stufe durch seinen pH-Wert (5,5), seinen reduzierten Gehalt an Phospholipiden (in der Größenordnung von 0,1 g/Liter) und seinen Gehalt an Veratrylalkohol (in der Größenordnung von 2,5 mM) unterscheidet.

5. Verfahren nach einem der Ansprüche 1 bis 4, dadurch **gekennzeichnet,** daß das Kulturmilieu der dritten Stufe sich von dem Kulturmilieu der zweiten Stufe durch seinen verringerten Gehalt an Glycerin (etwa 2,5 g/Liter), an Stickstoffquelle (insbesondere 0,46 g/Liter ± 0,04 g/Liter an Diammoniumtartrat) und an Hefeextrakt (0,25 g/Liter) unterscheidet.

6. Verfahren nach einem der Ansprüche 1 bis 5, dadurch **gekennzeichnet,** daß das Kulturmilieu der vierten Stufe sich von dem Kulturmilieu der dritten Stufe dadurch unterscheidet, daß es weder Hefeextrakt noch eine Stickstoffquelle noch eine Kohlenstoffquelle enthält.

7. Verfahren nach einem der Ansprüche 1 bis 6, dadurch **gekennzeichnet,** daß die erhaltenen Kulturen gewonnen werden und durch Filtration abgetrennt werden, und anschließend das erhaltene extrazelluläre Milieu konzentriert wird, um die gewünschte Ligninperoxidase zu gewinnen.


## Claims

1. A method of producing lignin peroxidase from a fungus known as *Phanerochaete chrysosporium*, in a culture medium containing activators and protectors of the enzyme, such as phospholipids and veratryl alcohol, which method is characterized in that it comprises:
   - a first step involving the culture of cells of *Phanerochaete chrysosporium* for an incubation period of 2 days, in a synthetic culture medium comprising salts of potassium, calcium and magnesium, trace elements (iron, zinc, manganese, copper), an appropriate source of nitrogen, a source of carbon consisting of glycerol, yeast extract, a source of phospholipids and a source of emulsified fatty acids, which first step takes place at an incubation temperature of 37°C ± 0.5°C, the culture medium being buffered at pH 6.5;
   - a second step involving culture of the mycelium formed during the first step, for a period of 3 days (from D3 to D5), in a culture medium which has been partially renewed (preferably renewed to the extent of 15%), to which veratryl alcohol - an activator and protector of lignin peroxidase production - has been added, together with phospholipids, and whose content of phospholipids has been reduced to 1/7 - 1/8 of what it was in the culture medium of the first step, which present culture medium does not contain emulsified fatty acids and is buffered at pH 5.5, this second step taking place at an incubation temperature of 30°C ± 0.5°C;
   - a third step involving culture at the optimum lignin peroxidase production, i.e. after 5 days, during which the whole of the culture medium of the second step is replaced with a synthetic culture medium analogous to that of the first step, which contains the same proportion of phospholipids and veratryl alcohol as the culture medium of the second step, and in which the yeast extract, the source of nitrogen and the source of carbon - consisting of glycerol - have been reduced to 1/4 of their content in the culture medium of the first step, these three components together forming a partial regeneration medium for the biomass, the incubation temperature being 30°C ± 0.5°C and the pH of the culture medium being 5.5;
- a fourth step consisting in continuing the culture with non-proliferating cells for 8 days (from D6 to D13), the culture medium being totally renewed every 24 hours and replaced with a medium not containing yeast extract, emulsified fatty acids or glycerol, but containing the activators and protectors of the enzyme production, consisting of veratryl alcohol and phospholipids, in the same proportions as the culture media of the second and third steps, the pH and the incubation temperature being the same as during the second and third steps; and
- a fifth step involving separation of the enzyme produced and, if necessary, purification of said enzyme.

2. A method according to Claim 1, characterized in that the source of phospholipids consists of soya azolectin.

3. A method according to Claim 1, characterized in that the culture medium of the first step has a content of nitrogen source of the order of 1.84 g/litre for a content of carbon source (glycerol) of the order of 10 g/litre, for a content of phospholipids (soya azolectin) of the order of 0.75 g/litre and for a content of yeast extract of 1 g/litre.

4. A method according to any one of Claims 1 to 3, characterized in that the culture medium of the second step differs from the culture medium of the first step by its pH (5.5), its reduced content of phospholipids (of the order of 0.1 g/litre) and its content of veratryl alcohol (of the order of 2.5 mM).

5. A method according to any one of Claims 1 to 4, characterized in that the culture medium of the third step differs from the culture medium of the second step by its reduced content of glycerol (about 2.5 g/litre), nitrogen source (in particular 0.46 g/litre ± 0.04 g/l of diammonium tartrate) and yeast extract (0.25 g/ litre).

6. A method according to any one of Claims 1 to 5, characterized in that the culture medium of the fourth step differs from the culture medium of the third step in that it does not contain yeast extract, a source of nitrogen or a source of carbon.

7. A method according to any one of Claims 1 to 6, characterized in that the cultures obtained are harvested and separated off by filtration, after which the extracellular medium obtained is concentrated in order to collect the desired lignin peroxidase.

FIG.1

Temps d'incubation (jours)

TEMPERATURE (°C)

ACTIVITE LIGNINASE (nkat/ml) (□)

BIOMASSE (g/l) (○)

EP 0 437 500 B1

FIG.2